# EUROPEAN PATENT APPLICATION

(11) **EP 4 424 714 A1**
(43) Date of publication of application: **04.09.2024**
(21) Application number: 21961885.7
(22) Date of filing: 29.10.2021
(51) Int. Cl.: C07K 19/00, C12N 15/62, C12N 5/10, A61K 39/00

(54) **CONDITION-CONTROLLED SPLICEABLE CHIMERIC ANTIGEN RECEPTOR MOLECULE AND APPLICATION THEREOF**

(71) Applicant: Shanghai Sinobay Biotechnology Co., Ltd., Shanghai 201506 (CN)
(72) Inventor: XU, Jianqing, Shanghai 201506 (CN); ZHANG, Xiaoyan, Shanghai 201506 (CN); LIAO, Qibin, Shanghai 201506 (CN); DING, Xiangqing, Shanghai 201506 (CN); WANG, Shiyu, Shanghai 201506 (CN)
(74) Representative: Potter Clarkson
(86) International application number: PCT/CN2021/127446
(87) International publication number: WO 2023/070527

(57) **Abstract**

A condition-controlled spliceable chimeric antigen receptor molecule and the use thereof. The spliceable chimeric antigen receptor molecule comprises an antigen recognition unit and a signal transduction unit; the antigen recognition unit comprises an antigen recognition domain, a transmembrane domain, a costimulatory signal domain, an N-terminal splicing domain, and a degrader; and the signal transduction unit comprises a conditional signal response domain, a C-terminal splicing domain, and a signaling domain. Such a condition-controlled spliceable system can achieve splicing of the two units and signaling under a tumor microenvironment signal. The antigen recognition unit can spontaneously/be induced to degrade, thus reducing retention in normal tissues. A signaling unit can respond to a specific condition signal of a tumor microenvironment, and has the characteristics of low expression in a normal tissue environment and high expression in the tumor microenvironment. The condition-controlled spliceable system can achieve preparation of drugs and precise treatment for solid tumors by grafting different functional genes.

## Description

### Technical Field

The present invention belongs to the field of biomedical technology, and particularly, relates to a condition-controlled spliceable chimeric antigen receptor molecule and a coding nucleic acid molecule, a vector and a host cell thereof, as well as their use in the treatment of hypoxic diseases.

### Background Art

Cancer immunotherapy involves monoclonal antibodies, vaccines, gene therapy, cell therapy and other therapies, wherein monoclonal antibodies targeting tumor antigens, immune checkpoint inhibitors, T cell receptor T cells (TCR-T cells), chimeric antigen receptor T cells (CAR-T cells) and other immunotherapies have shown remarkable anti-tumor effects in clinical studies. However, in terms of cell therapy, an off-target effect brought by the absence of tumor-specific antigens may often lead to fatal side effects (Morgan RA, Yang JC, Kitano M, et al. Case report of a serious adverse event following the administration of T cells transduced with a chimeric antigen receptor recognizing ERBB2 [J]. Mol Ther. 2010, 18: 843-851), thus greatly limiting the clinical application of the cell therapy in solid tumors. Therefore, it is necessary to further develop controllable and spatiotemporally specific cell immunotherapy technologies in order to reduce the damages to normal tissues and improve the specificity for the treatment of tumor lesions.

Previous studies have reported a modular CAR design in which an intact CAR molecule is divided by researchers into two modules: an antigen recognition module located on a cell membrane to recognize antigens on the surfaces of tumor cells, and an intracellular signaling module. The activation of modular CAR is based on two conditions. One condition is that an engineered T cell should recognize the antigens on the surfaces of the tumor cells through the antigen recognition module, and the engineered T cell binds to the tumor cells, but cannot be activated by itself, and the tumor cells can be killed once the antigen recognition module and the signaling module are coupled to transmit an activation signal, only in the presence of external small molecule compounds (Wu CY, Roybal KT, Puchner EM, Onuffer J, Lim WA. Remote control of therapeutic T cells through a small molecule-gated chimeric receptor [J]. 2 Science. 2015, 350(6258): aab4077). However, the activation of the above-mentioned modular CAR is strictly dependent on the addition of the external small molecule compounds, and appropriate timing for the application and deactivation of the external small molecule compounds poses a great challenge to clinical applications.

Modern medical research has found that hypoxia is closely related to arthritis, diabetic retinopathy, ischemic heart disease, stroke and many other diseases, especially solid tumors. Hypoxia is a common characteristic signal for many solid tumors due to insufficient blood supply caused by abnormal vascular structures in solid tumors and excessive proliferation of the tumor cells, which creates a relatively hypoxic tumor microenvironment, with an oxygen level in tumor tissues being often less than 2% (Brown JM, Wilson WR. Exploiting tumour hypoxia in cancer treatment [J]. Nat Rev Cancer, 2004, 4: 437-447). Therefore, how to strictly control the activation drive of the modular CAR by using a difference between a tumor microenvironment and a normal tissue microenvironment as an influence factor, so as to realize its clinical applications in tumor treatment, such that the inconvenient and inaccurate problems caused by the activation of the modular CAR driven by the external small molecule compounds in the prior art can be solved.

### Summary of the Invention

In view of the above-mentioned problems existing in the prior art, an object of the present invention is to provide a condition-controlled spliceable chimeric antigen receptor (CAR) molecule and the use thereof in the treatment of hypoxic diseases such as solid tumors, especially in a CAR-T cell therapy of solid tumors.

In the present invention, some terms are described or defined as follows.

The term "spliceable" refers to the fact that protein molecules may be cleaved and then rejoined.

The term "antigen recognition unit" refers to a protein molecule that can specifically bind to and recognize a target antigen.

The term "signal transduction unit" refers to a protein molecule that can transmit signals.

The term "antigen recognition domain" refers to a functional domain that can specifically recognize a target antigen, generally including a single-chain fragment variable (scFv), a single domain antibody (sdAb), and an extracellular end of a receptor.

The term "transmembrane domain" refers to a region in a protein sequence across a cell membrane.

The term "costimulatory signal domain" refers to a domain in which different costimulatory molecules and their ligands expressed on the surface of an immune cell involved in adaptive immunity bind with each other to produce a costimulatory signal, the domain generally being a costimultory signal domain selected from CD27, CD28, 4-1BB, OX40, ICOS and other costimulatory molecules.

The term "N-terminal splicing domain" refers to an N-terminal domain of an intein, which may be spliced with a C-terminal splicing domain.

The term "degrader" refers to a specific amino acid sequence that may be recognized by an intracellular protease to mediate the degradation and clearance of a target protein.

The term "conditional signal response domain" refers to a specific amino acid sequence that may respond to specific conditional signals and regulate the degradation and enrichment of a target protein, the specific conditional signals including oxygen content, illumination and temperature.

The term "C-terminal splicing domain" refers to a C-terminal domain of an intein, which may be spliced with the N-terminal splicing domain.

The term "signaling domain" refers to a signaling domain of a T cell receptor containing a plurality of immunoreceptor tyrosine-based activation motifs (ITAMs), including CD3γ, CD3δ, CD3ε, and CD3ζ.

The term "hypoxic disease" refers to a disease in which tissue oxygen content is less than 1%, particularly a solid tumor.

The objects of the present invention are achieved by the following technical solutions.

In a first aspect, the present invention provides a condition-controlled spliceable chimeric antigen receptor molecule comprising an antigen recognition unit and a signal transduction unit, wherein the antigen recognition unit includes an antigen recognition domain, a transmembrane domain, a costimulatory signal domain, an N-terminal splicing domain, and a degrader; and the signal transduction unit includes a conditional signal response domain, a C-terminal splicing domain, and a signaling domain.

The condition-controlled spliceable chimeric antigen receptor molecule provided by the present invention can respond to specific conditional signals, such as hypoxia, thereby achieving splicing of the antigen recognition unit and the signal transduction unit. The antigen recognition unit can spontaneously/be induced to degrade, thus reducing retention in normal tissues. The signaling unit can respond to specific conditional signals, such as hypoxia, and has the characteristics of low expression in a normal tissue environment and enrichment in a tumor microenvironment.

In the chimeric antigen receptor molecule according to the present invention, an antigen to which the antigen recognition domain binds may be one or more selected from CD47, AXL, EGFR, CD7, CD24, FAP, CD147, HER2, ROR1, ROR2, CD133, EphA2, CD171, CEA, EpCAM, TAG72, IL-13Rα, EGFRvIII, GD2, FRα, PSCA, PSMA, GPC3, CAIX, Claudin18.2, VEGFR2, PD-L1, MSLN, MUC1, c-Met, B7-H3 or TROP2 antigen. Preferably, the antigen to which the antigen recognition domain binds is the CD47 antigen.

In the chimeric antigen receptor molecule according to the present invention, the transmembrane domain may be one or more selected from the CD3ζ, CD4, CD8, CD28 or CD137/4-1BB transmembrane domain. Preferably, the transmembrane domain is the CD28 transmembrane domain.

In the chimeric antigen receptor molecule according to the present invention, the costimulatory domain in the antigen recognition unit may be one or more selected from CD2, CD27, CD28, CD40, OX40, CD137/4-1BB, TLR1, TLR2, TLR3, TLR4, TLR5, TLR6, TLR7, TLR8, TLR9, TLR10, TLR11 or Dap10 costimulatory domain. Preferably, the costimulatory domain in the antigen recognition unit is one or more selected from the CD28 or CD137/4-1BB costimulatory structural domain.

In the chimeric antigen receptor molecule according to the present invention, the N-terminal splicing domain may be one or more selected from a protein intron or a SpyTag/SpyCatcher self-assembler. Preferably, the N-terminal splicing domain is a protein intron Int^{N}.

In the chimeric antigen receptor molecule according to the present invention, the degrader may be one or more selected from a dihydrofolate reductase (DHFR), an estrogen receptor (ER), a Salmonella type III secretion system effector protein (SopE), a plant hormone-inducible protein degrader, an unstable domain (AD) or a photosensitive protein degrader. Preferably, the degrader is s one or more elected from the estrogen receptor or the Salmonella type III secretion system effector protein. Most preferably, the degrader is a mutant estrogen receptor (ERm).

In the chimeric antigen receptor molecule according to the present invention, the conditional signal response domain may be one or more selected from an oxygen-dependent degradation domain (ODD), a temperature-sensitive domain, a pH-sensitive domain, a photosensitive domain or an inflammatory cytokine response domain. Preferably, the conditional signal response structural domain is the oxygen-dependent degradation structural domain.

In the chimeric antigen receptor molecule according to the present invention, the C-terminal splicing domain may be one or more selected from a protein intron or a SpyTag/SpyCatcher self-assembler. Preferably, the C-terminal splicing domain is a protein intron Int^{C}.

In the chimeric antigen receptor molecule according to the present invention, the signaling domain may be one or more selected from a CD3ξ, FcyRIII, FcεRI or Fc receptor signaling domain or a signaling molecule carrying an immunoreceptor tyrosine-based activation motif (ITAM). Preferably, the signaling domain is the CD3ξ signaling domain.

In the chimeric antigen receptor molecule according to the present invention, preferably, the signal transduction unit further includes a costimulatory signal domain. More preferably, the costimulatory signal domain in the signal transduction unit is one or more selected from CD2, CD27, CD28, CD40, OX40, CD137/4-1BB, TLR1, TLR2, TLR3, TLR4, TLR5, TLR6, TLR7, TLR8, TLR9, TLR10, TLR11 or Dap10 costimulatory signal domain.

According to a preferred embodiment of the present invention, an amino acid sequence of the antigen recognition unit is shown in SEQ NO: 1 or SEQ NO: 2.

According to a preferred embodiment of the present invention, an amino acid sequence of the signal transduction unit is shown in SEQ NO: 3.

In a second aspect, the present invention provides a nucleic acid molecule that codes the chimeric antigen receptor molecule according to the present invention.

In the nucleic acid molecule of the present invention, preferably, a nucleotide sequence coding the signal transduction unit also includes a nucleotide sequence coding a conditional signal response element. Preferably, the conditional signal response element is one or more selected from a hypoxia response element (HRE), a temperature-sensitive element, a pH-sensitive element, a photosensitive element or an inflammatory cytokine response element. More preferably, the conditional signal response element is the hypoxia response element (HRE). The signal transduction unit may further enhance a response capability of a conditional signal at a nucleic acid level under the regulation of a nucleotide sequence as the hypoxia response element.

According to a preferred embodiment of the present invention, a nucleotide sequence coding the signal transduction unit is shown in SEQ NO: 4.

According to a preferred embodiment of the present invention, a nucleotide sequence of the nucleic acid molecule is shown in SEQ NO: 5 or 6.

In a third aspect, the present invention provides a vector, including the nucleic acid molecule according to the present invention.

The vector according to the present invention may be one or more selected from a plasmid, a retroviral vector, a lentiviral vector, an adenoviral vector, an adeno-associated viral vector, a vaccinia virus vector, a herpes simplex virus vector, a forest encephalitis virus vector, a poliovirus vector, a Newcastle disease virus vector, or a transposon. Preferably, the vector is the lentiviral vector.

In a fourth aspect, the present invention provides a genetically engineered host cell, including the exogenous nucleic acid molecule according to the present invention that is integrated in its chromosome, or including the vector according to the present invention.

The host cell according to the present invention may be selected one or more from an isolated human-derived cell or a genetically engineered immune cell.

Preferably, the isolated human-derived cell is one or more selected from an embryonic stem cell, an umbilical cord blood-derived stem cell, an induced pluripotent stem cell, a hematopoietic stem cell, a mesenchymal stem cell, an adipose-derived stem cell, a T cell, an NK cell, an NKT cell or a macrophage.

Preferably, the genetically engineered immune cell is one or more selected from a genetically engineered T cell, NK cell, NKT cell or macrophage. More preferably, the genetically engineered immune cell is the genetically engineered T cell.

According to a preferred embodiment of the present invention, the genetically engineered immune cell is one or more selected from a chimeric antigen receptor T cell (CAR-T cell), a chimeric antigen receptor NK cell (CAR-NK cell), a chimeric antigen receptor NKT cell (CAR-NKT cell), a chimeric antigen receptor macrophage (CAR-mø) or a T cell receptor T cell (TCR-T cell). According to a more preferred embodiment of the present invention, the genetically engineered immune cell is a chimeric antigen receptor T cell.

The genetically engineered host cell according to the present invention can drive the splicing of a modular CAR in a hypoxic environment after transduction of the nucleic acid molecule according to the present invention or transfection of the vector according to the present invention.

The present invention further provides a method for preparing the genetically engineered host cell according to the present invention, including: introducing into the host cell the nucleic acid molecule and/or the vector according to the present invention.

In the method according to the present invention, the introduction may be transfection or transduction.

In a fifth aspect, the present invention provides use of the condition-controlled spliceable chimeric antigen receptor molecule, the nucleic acid molecule, the vector, or the genetically engineered host cell according to the present invention in the preparation of a medicament for cell immunotherapy.

Correspondingly, the present invention provides a cellular immunotherapy method, including the steps of:
introducing into a host cell the nucleic acid molecule and/or the vector according to the present invention, and administrating to a subject in need thereof a therapeutically effective amount of the host cell; or
administrating to the subject in need thereof a therapeutically effective amount of the genetically engineered host cell according to the present invention.

Preferably, the introduction may be performed simultaneously, in a chronological order, or in sequence.

Preferably, in the use or method according to the present invention, the medicament or method for cell immunotherapy is used for the treatment of hypoxic diseases. Preferably, the hypoxic disease is a cancer. More preferably, the cancer is a solid tumor, such as one or more of neuroblastoma, lung cancer, breast cancer, esophageal cancer, gastric cancer, fiver cancer, cervical cancer, ovarian cancer, kidney cancer, pancreatic cancer, nasopharyngeal cancer, small bowel cancer, large bowel cancer, colorectal cancer, bladder cancer, bone cancer, prostate cancer, thyroid cancer or brain cancer.

The inventors have found under research that the activation of the modular CAR driven by an intrinsic characteristic signal of a tumor microenvironment is more valuable for clinical applications. Hypoxic microenvironment signals for tumors are expected to be used in the development of new technologies for the treatment of hypoxic diseases such as solid tumors, and the activation of the modular CAR driven by the hypoxic microenvironment signals can improve the specificity of tumor treatment, thereby avoiding or reducing the damages to normal tissues.

The condition-controlled spliceable chimeric antigen receptor molecule provided by the present invention includes an antigen recognition unit and a signal transduction unit, which may respond to specific conditional signals (e.g., hypoxia) and thereby achieve splicing of the antigen recognition unit and the signal transduction unit. The following three advantages are reflected:
1) the antigen recognition unit can spontaneously/be induced to degrade, thus reducing retention in normal tissues and damages to normal tissues;
2) the signaling unit may respond to a specific condition signal (e.g., hypoxia), and has the characteristics of low expression in a normal tissue environment and enrichment in the tumor microenvironment; and
3) the condition-controlled modular CAR splicing achieves specific activation in the tumor microenvironment, and effectively reduces on-target off-tumor toxic and side effects caused by CAR-T cells targeting non-tumor tissues.

It should be understood that the above technical features of the present invention and the technical features specifically described below (e.g., in examples) may be combined with each other within the scope of the present invention, thereby constituting a new or preferred technical solution. Due to limited space, these technical features are not repeated one by one.

### Brief Description of the Drawings

The embodiments of the present invention will be described in detail below with reference to the accompanying drawings. In drawings:

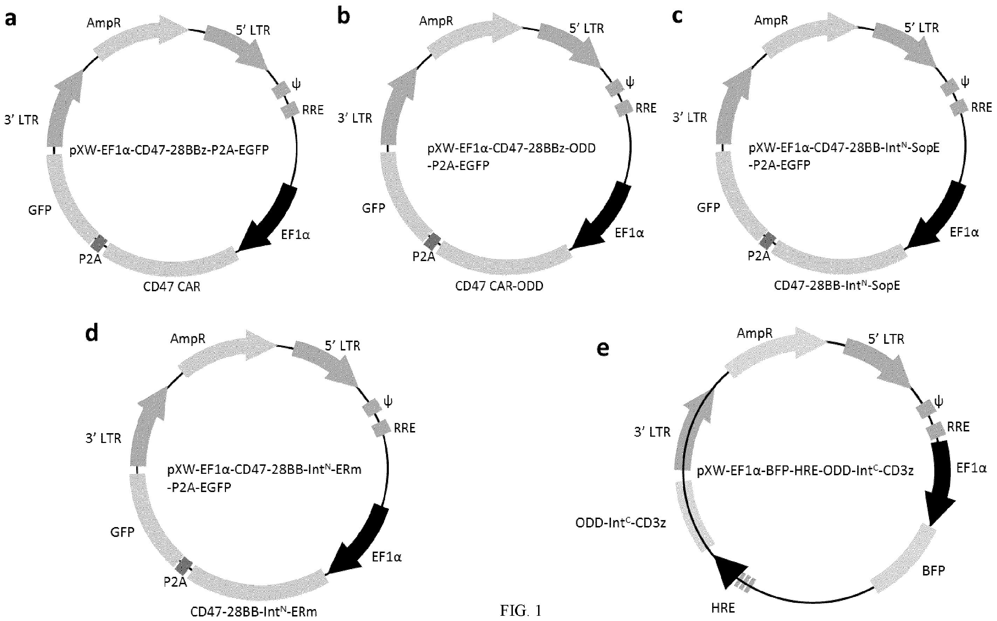

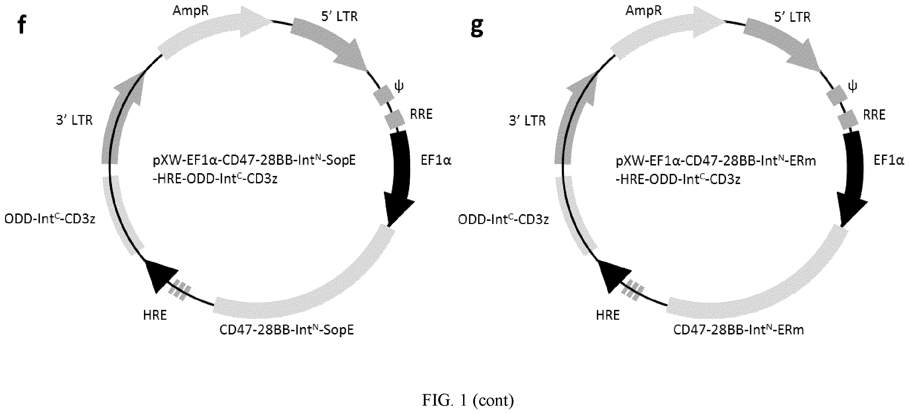

FIG. 1 shows lentiviral expression plasmid profiles of conventional CD47 CAR (CD47-28BBZ, FIG. 1a), hypoxia-regulated CD47 CAR-ODD fused with an oxygen-dependent degradation domain ODD (CD47-28BBz-ODD, FIG. 1b), an antigen recognition unit 1 (CD47-28BB-Int^{N}-SopE, FIG. 1c), an antigen recognition unit 2 (CD47-28BB-Int^{N}-ERm, FIG. 1d), a hypoxia-regulated signal transduction unit (ODD-Int^{C}-CD3z, FIG. 1e), a two-in-one condition-controlled (hypoxia-regulated) spliceable system 1 (CD47-28BB-Int^{N}-SopE-HRE-ODD-Int^{C}-CD3z, FIG. 1f) and a two-in-one condition-controlled (hypoxia-regulated) spliceable system 2 (CD47-28BB-Int^{N}-ERm-HRE-ODD-Int^{C}-CD3z, FIG. 1g).

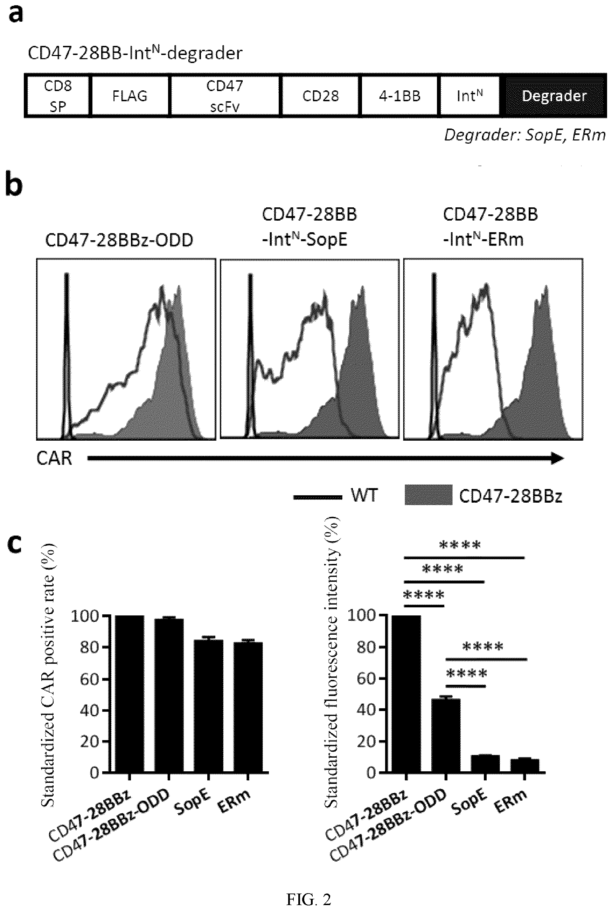

FIGS. 2a-c show the spontaneous degradation of the antigen recognition unit 1 (CD47-28BB-Int^{N}-SopE) and the antigen recognition unit 2 (CD47-28BB-Int^{N}-ERm). FIG. 2a shows a structure and composition of the antigen recognition unit; FIG. 2b shows flow cytometry detection results of CD47 CAR fused with an oxygen-dependent degradation domain ODD alone, an antigen recognition unit 1 fused with a degrader SopE and an antigen recognition unit 2 fused with a degrader ERm on a 293T cell membrane; and FIG. 2c shows a statistical result of flow cytometry detection data in FIG. 2b, wherein the spontaneous degradation capabilities of the degraders SopE and ERm are significantly superior to that of the oxygen-dependent degradation domain ODD, and ERm has the best degradation capability.

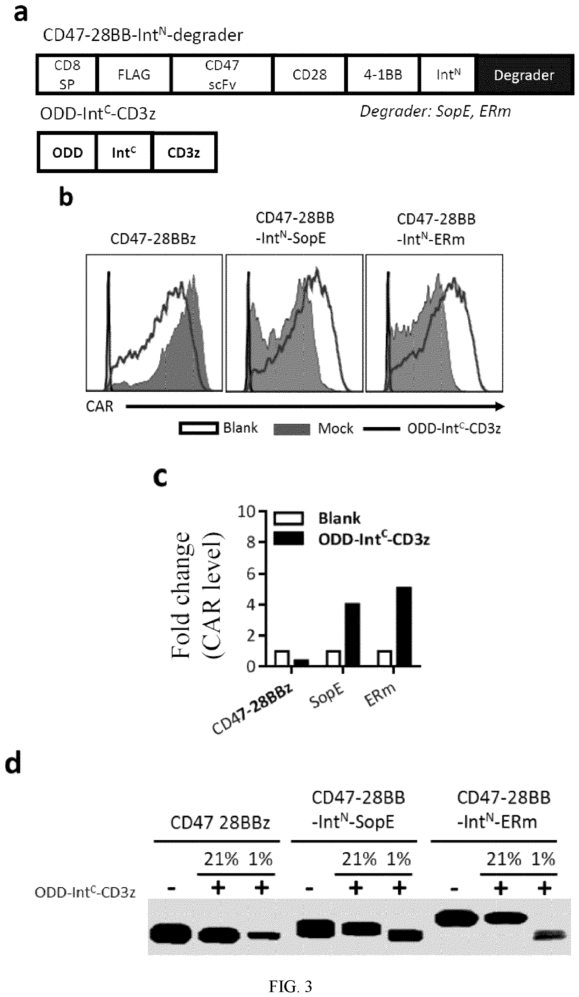

FIGS. 3a-d show splicing activities of the antigen recognition unit 1 (CD47-28BB-Int^{N}-SopE) and the antigen recognition unit 2 (CD47-28BB-Int^{N}-ERm). FIG. 3a shows structures and compositions of degrader-fused CAR and a hypoxia-regulated signaling unit ODD-IntC-CD3z; FIG. 3b shows CAR expression levels of an engineered 293T cell that integrates the CAR and the hypoxia-regulated signaling unit ODD-IntC-CD3z, and an engineered 293T cell that integrates the degrader-fused CAR and the hypoxia-regulated signaling unit ODD-IntC-CD3z under a hypoxic condition, wherein Blank is a flow cytometry detection result of a negative 293T cell, and Mock indicates an engineered 293T cell without the hypoxia-regulated signaling unit ODD-IntC-CD3z, that is, an engineered 293T cell that is transduced with a no-load virus; FIG. 3c shows an induction fold of CAR expression levels after splicing under the hypoxic condition; and FIG. 3d shows results of western blotting detection for three genetically engineered 293T cells which integrate the hypoxia-regulated signaling unit ODD-IntC-CD3z at the same time and are collected after being cultured for 24 h under a normoxic condition (21% O₂) and a hypoxic condition (1% O₂) respectively.

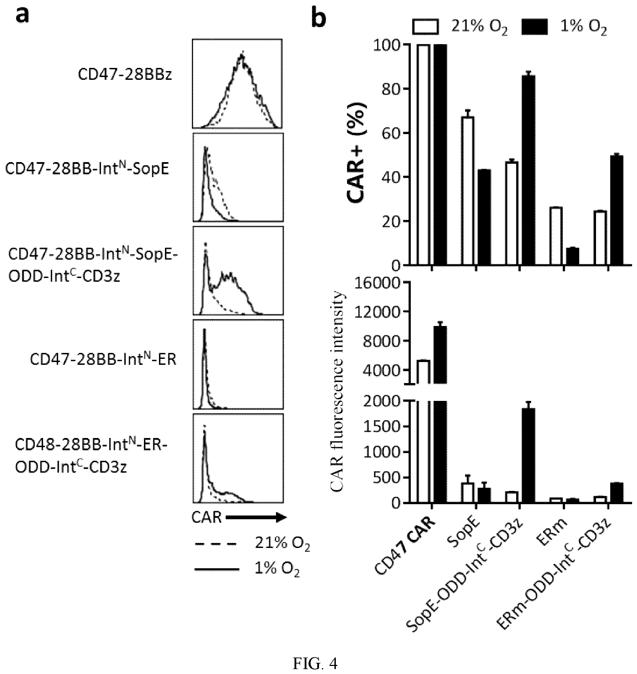

FIG. 4 shows flow cytometry detection results of CAR molecules on the cell membranes of engineered T cells that transduce the CD47 CAR (CD47-28BBZ, z representing a CD3ζ signaling domain), the antigen recognition unit 1 (CD47-28BB-Int^{N}-SopE), the two-in-one condition-controlled (hypoxia-regulated) spliceable system 1 (CD47-28BB-Int^{N}-SopE -HRE-ODD-Int^{C}-CD3z), the antigen recognition unit 2 (CD47 -28BB-Int^{N}-ERm) and the two-in-one condition-controlled (hypoxia-regulated) spliceable system 2 (CD47-28BB-Int^{N}-ERm-HRE-ODD-Int^{C}-CD3z). FIG. 4b shows a statistical result of the flow cytometry detection data in FIG. 4a.

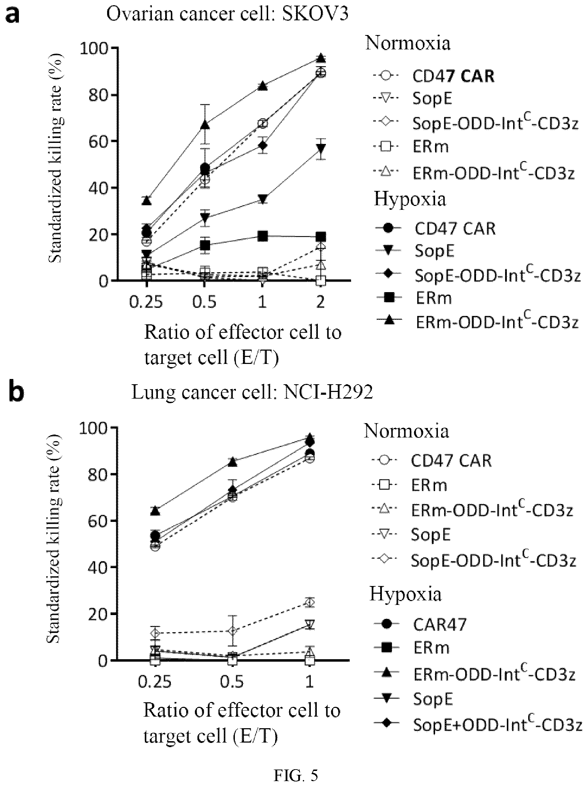

FIG. 5 shows normoxia- and hypoxia-dependent cell killing results of engineered T cells that transduce the CD47 CAR (CD47-28BBz), the antigen recognition unit 1 (CD47-28BB-Int^{N}-SopE), the two-in-one condition-controlled (hypoxia-regulated) spliceable system 1 (CD47-28BB-Int^{N}-SopE -HRE-ODD-Int^{C}-CD3z), the antigen recognition unit 2 (CD47 -28BB-Int^{N}-ERm) and the two-in-one condition-controlled (hypoxia-regulated) spliceable system 2 (CD47-28BB-Int^{N}-ERm-HRE-ODD-Int^{C}-CD3z). Target cells in FIG. 5a are of an ovarian cancer tumor cell line SKOV3; and target cells in FIG. 5b are of a lung cancer tumor cell line NCI-H292.

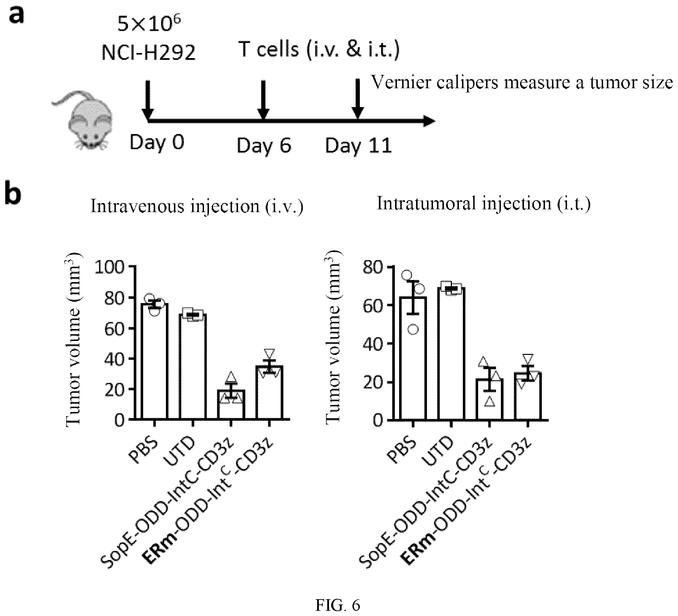

FIG. 6 shows in-vivo antitumor activities of the engineered T cells that transduce the two-in-one condition-controlled (hypoxia-regulated) spliceable system 1 (CD47-28BB-Int^{N}-SopE -HRE-ODD-Int^{C}-CD3z) and the two-in-one condition-controlled (hypoxia-regulated) spliceable system 2 (CD47-28BB-Int^{N}-ERm-HRE-ODD-Int^{C}-CD3z). FIG. 6a is an experimental flow chart; and FIG. 6b is a statistical result of tumor sizes after infusion of the engineered T cell for 30 days.

### Mode of Carrying Out the Invention

The present invention is further described below in conjunction with specific examples, and the advantages and characteristics of the present invention will be clearer with the description. The following examples are used to illustrate the present invention, but not to limit the scope of the present invention.

Experimental methods used in the following examples were conventional experimental methods in the art unless otherwise specified. Experimental materials used in the following examples, unless otherwise specified, were purchased from conventional biochemical reagent sales companies, wherein:
DMEM medium and RPMI1640 medium were purchased from Corning, and lymphocyte medium X-VIVO 15 was purchased from Lonza.

T cell growth medium, which was composed of a basal medium and cytokines, was prepared with reference to the Chinese invention patent CN201910163391.1. The basal medium was a lymphocyte medium X-VIVO 15, and the cytokines, i.e., 5 ng/mL IL-7, 10 ng/mL IL-15 and 30 ng/mL IL-21 were added. The cytokines IL-7 and IL-15 were purchased from R&D, and IL-21 was purchased from Nearshore Protein Technology Co., Ltd.

Fetal bovine serum was purchased from BI Inc.

ATurboFect Transfection Kit was purchased from Thermo Fisher Scientific.

A Lenti-X lentiviral concentrate reagent was purchased from Takara, Inc.

Gene synthesis was completed by Shanghai Generay Bioengineering Co., Ltd.

Blank lentiviral expression plasmids (pXW-EF1α-MCS-P2A-EGFP and pXW-EF1α-BFP-MCS1-HRE-MCS2) were derived from a commercial plasmid ABpCCLsin-EF1α-MCS purchased from Kanglin Biotechnology (Hangzhou) Co., Ltd. A green fluorescent protein gene and a blue fluorescent protein gene were respectively recombined on this commercial plasmid to obtain the blank lentiviral expression plasmids. A packaging plasmid psPAX2 and an envelope plasmid PMD2.G were purchased from Addgene.

Stable 3 chemically competent cells were purchased from Shanghai Weidi Biotechnology Co., Ltd.

An endotoxin-free plasmid miniprep kit and an endotoxin-free plasmid midiprep kit were purchased from OMEGA and Macherey Nagel, respectively.

A luciferase substrate was purchased from Promega Biotechnology Co., Ltd.

293T cells, A549 lung cancer cells, SKOV3 ovarian cancer cells, and NCI-H292 lung cancer cells were purchased from ATCC in the United States. SKOV3-luc and NCI-H292-luc, which stably integrated a firefly luciferase gene respectively, were obtained by transduction of SKOV3 ovarian cancer cells and NCI-H292 lung cancer cells using a lentivirus carrying the firefly luciferase gene.

Severe combined immuno-deficient mice (B-NDG) were purchased from Biocytogen (Jiangsu) Gene Biotechnology Co., Ltd.

### Example 1: Composition of condition-controlled spliceable chimeric antigen receptor molecule

An amino acid sequence of the antigen recognition unit was shown as SEQ NO: 1 or SEQ NO: 2. SEQ NO: 1 or SEQ NO: 2 was composed of sequences of a CD47 antigen recognition domain, a CD28 transmembrane domain, a CD28 and 4-1BB costimulatory signal domain, an N-terminal splicing domain and a degrader, respectively. The amino acid sequences of the CD47 antigen recognition domain, the CD28 transmembrane domain, the CD28 and 4-1BB costimulatory signal domain, the N-terminal splicing domain and the degrader were specifically shown in Table 1.

**Table 1 Amino acid sequence of antigen recognition unit**

| Name | Amino acid sequence |
|---|---|
| CD47 antigen recognition domain | MALPVTALLLPLALLLHAARPDYKDDDDKEVQLVESGGGLVQP GGSLRLSCAASGFTFSGYGMSWVRQAPGKGLEWVATITSGGTY TYYPDSVKGRFTISRDNAKNSLYLQMNSLRAEDTAVYYCARSL AGNAMDYWGQGTLVTVSSGGGGSGGGGSGGGGSEIVLTQSPA TLSLSPGERATLSCRASQTISDYLHWYQQKPGQAPRLLIKFASQS ISGIPARFSGSGSGTDFTLTISSLEPEDFAVYYCQNGHGFPRTFGQ GTKVEIK |
| CD28 transmembrane domain | FWVLVVVGGVLACYSLLVTVAFIIFWV |
| CD28 and 4-1BB costimulatory signal domain | RSKRSRLLHSDYMNMTPRRPGPTRKHYQPYAPPRDFAAYRSRF SVVKRGRKKLLYIFKQPFMRPVQTTQEEDGCSCRFPEEEEGGCE L |
| N-terminal splicing domain | TRSGYCLDLKTQVQTPQGMKEISNIQVGDLVLSNTGYNEVLNV FPKSKKKSYKITLEDGKEIICSEEHLFPTQTGEMNISGGLKEGMC LYVKE |
| Degrader SopE | TKITLSPQNFRIQKQETTLLKEKSTEKNSLAKSILAVKNHFIELRS KLSERFISHKNTESSATHFHRGSASEGRAVLTNKVVKDFMLQTL NDIDIRGSA |
| Degrader ERm | SLALSLTADQMVSALLDAEPPILYSEYDPTRPFSEASMMGLLTN LADRELVHMINWAKRVPGFVDLALHDQVHLLECAWMEILMIGL VWRSMEHPGKLLFAPNLLLDRNQGKCVEGGVEIFDMLLATSSR FRMMNLQGEEFVCLKSIILLNSGVYTFLSSTLKSLEEKDHIHRVL DKITDTLIHLMAKAGLTLQQQHQRLAQLLLILSHIRHMSSKRME HLYSMKCKNVVPLSDLLLEMLDAHRL |

An amino acid sequence of the signal transduction unit was shown in SEQ NO: 3. SEQ NO: 3 was composed of sequences of a hypoxic condition signal response domain (oxygen-dependent degradation domain ODD), a C-terminal splicing domain and a CD3ζ signaling domain. The amino acid sequences of the hypoxic condition signal response domain (oxygen-dependent degradation domain ODD), the C-terminal splicing domain and the CD3ζ signaling domain were specifically shown in FIG. 2.

**Table 2 Amino acid sequence of signal transduction unit**

| Name | Amino acid sequence |
|---|---|
| Hypoxic condition signal response domain (oxygen-dependent degradation domain ODD) | MSEDTSSLFDKLKKEPDALTLLAPAAGDTIISLDFGSNDT ETDDQQLEEVPLYNDVMLPSPNEKLQNINLAMSPLPTA ETPKPLRSSADPALNQEVALKLEPNPESLELSFTMPQIQD QTPSPSDGSTRQSSPEPNSPSEYCFYVDSDMVNEFKLEL VEKLFAEDTEAKNPFSTQDTDLDLEMLAPYIPMDDDFQ LRSFDQLSPLESSSASPESASPQSTVTVFQ |
| C-terminal splicing domain | LKKILKIEELDERELIDIEVSGNHLFYANDILTHNSSSSDV |
| CD3ζ signaling domain | RVKFSRSADAPAYQQGQNQLYNELNLGRREEYDVLDK RRGRDPEMGGKPQRRKNPQEGLYNELQKDKMAEAYSE IGMKGERRRGKGHDGLYQGLSTATKDTYDALHMQALP PR |

A nucleotide sequence of the signal transduction unit containing a hypoxia response element was shown in SEQ NO: 4. SEQ NO: 4 was composed of sequences of the hypoxic condition signal response domain (oxygen-dependent degradation domain ODD), the C-terminal splicing domain and the CD3ζ signaling domain. The nucleotide sequences of the hypoxic condition signal response domain (oxygen-dependent degradation domain ODD), the C-terminal splicing domain, the CD3ζ signaling domain and the hypoxia response element were specifically shown in FIG. 3.

**Table 3 Nucleotide sequence of antigen transduction unit**

| Name | Nucleotide sequence |
|---|---|
| Hypoxic condition signal response domain (oxygen-dependent degradation domain ODD) | ATGAGCGAGGACACCAGCAGCCTGTTCGACAAGCTG AAGAAGGAGCCCGACGCCCTGACCCTGCTGGCCCCT GCTGCTGGAGACACCATCATCTCCCTGGACTTCGGCA GCAACGACACCGAGACCGACGACCAGCAGCTGGAGG AGGTGCCCCTGTACAACGACGTGATGCTGCCCTCTCC CAACGAAAAACTGCAGAACATCAACCTGGCTATGAG CCCCCTGCCCACCGCCGAAACACCAAAACCCCTGAG ATCCAGCGCCGACCCCGCCCTGAACCAGGAAGTGGC CCTGAAACTGGAACCCAACCCCGAGAGCCTGGAGCT GAGCTTCACCATGCCCCAGATCCAGGACCAGACCCCC AGCCCCAGCGACGGAAGCACCAGACAGAGCAGCCCC GAGCCTAACTCCCCCAGCGAATACTGCTTCTATGTGG ACAGCGACATGGTGAACGAGTTCAAGCTGGAGCTGG TGGAAAAACTGTTCGCCGAGGACACAGAAGCCAAAA ACCCCTTCAGCACCCAGGACACAGACCTGGACCTGG AGATGCTGGCCCCCTACATCCCCATGGACGACGACTT CCAGCTGAGATCCTTCGACCAGCTGAGCCCCCTGGAA AGCAGCAGCGCCTCCCCCGAATCAGCCAGCCCCCAG AGCACCGTGACCGTGTTCCAG |
| C-terminal splicing domain | CTGAAAAAGATCCTGAAGATCGAGGAGCTGGACGAG CGGGAACTGATCGACATCGAAGTGTCCGGAAACCAC CTGTTCTACGCCAACGACATCCTGACACACAATAGCA GCAGCAGCGACGTG |
| CD3ζ signaling domain | AGAGTGAAATTCAGCAGAAGCGCCGACGCCCCCGCC TACCAGCAGGGACAGAATCAGCTGTACAACGAACTG AACCTGGGCAGAAGGGAGGAATACGACGTGCTGGAC AAGAGGAGAGGAAGGGACCCCGAGATGGGAGGAAA ACCACAGAGAAGAAAGAACCCACAGGAGGGACTGTA CAACGAGCTGCAGAAGGACAAGATGGCCGAGGCCTA CAGTGAAATTGGCATGAAGGGAGAGAGAAGAAGAGG AAAGGGACACGACGGCCTGTACCAGGGCCTGAGCAC CGCTACCAAGGACACATACGACGCCCTGCACATGCAG GCCCTGCCACCAAGA |
| Hypoxia response element | CCACAGTGCATACGTGGGCTCCAACAGGTCCTCTTCC ACAGTGCATACGTGGGCTCCAACAGGTCCTCTTCCAC AGTGCATACGTGGGCTCCAACAGGTCCTCTTCCACAG TGCATACGTGGGCTCCAACAGGTCCTCTTCCACAGTG CATACGTGGGCTCCAACAGGTCCTCTT |

A nucleotide sequence of the condition-controlled spliceable chimeric antigen receptor molecule was shown in SEQ NO: 5 or SEQ NO: 6. SEQ NO: 5 was composed of a nucleic acid coding sequence of the CD47 antigen recognition domain, a nucleic acid coding sequence of the CD28 transmembrane domain, a nucleic acid coding sequence of the CD28 and 4-1BB costimulatory signal domain, a nucleic acid coding sequence of the N-terminal splicing domain, a nucleic acid coding sequence of the degrader SopE, the hypoxia response element (HRE), a nucleic acid coding sequence of the hypoxic condition signal response domain (oxygen-dependent degradation domain ODD), a nucleic acid coding sequence of the C-terminal splicing domain and a nucleic acid coding sequence of the CD3ζ signaling domain. SEQ NO: 6 was composed of the nucleic acid coding sequence of the CD47 antigen recognition domain, the nucleic acid coding sequence of the CD28 transmembrane domain, the nucleic acid coding sequence of the CD28 and 4-1BB costimulatory signal domain, the nucleic acid coding sequence of the N-terminal splicing domain, a nucleic acid coding sequence of the degrader ERm, the hypoxia response element (HRE), the nucleic acid coding sequence of the hypoxic condition signal response domain (oxygen-dependent degradation domain ODD), the nucleic acid coding sequence of the C-terminal splicing domain and the nucleic acid coding sequence of the CD3ζ signaling domain. The nucleic acid coding sequence of the CD47 antigen recognition domain, the nucleic acid coding sequence of the CD28 transmembrane domain, the nucleic acid coding sequence of the CD28 and 4-1BB costimulatory signal domain, the nucleic acid coding sequence of the N-terminal splicing domain, the nucleic acid coding sequence of the degrader SopE/ERm, the hypoxia response element (HRE), the nucleic acid coding sequence of the hypoxic condition signal response domain (oxygen-dependent degradation domain ODD), the nucleic acid coding sequence of the C-terminal splicing domain and the nucleic acid coding sequence of the CD3ζ signaling domain were specifically shown in FIG. 4.

**Table 4 Nucleotide sequence of condition-controlled spliceable chimeric antigen receptor molecule**

| Name | Nucleotide sequence |
|---|---|
| CD47 antigen recognition domain | ATGGCTCTGCCAGTGACAGCTCTCCTCCTCCCACTC GCCCTGCTGCTGCACGCCGCTAGACCTGACTACAAG GACGACGACGACAAGGAGGTGCAGCTGGTGGAGTC TGGGGGAGGCTTGGTCCAGCCTGGGGGGTCCCTGA GACTCTCCTGTGCAGCCTCTGGATTCACCTTTAGTGG CTATGGCATGAGCTGGGTCCGCCAGGCTCCAGGGAA GGGGCTGGAGTGGGTGGCCACCATAACTAGTGGTGG AACTTACACCTACTATCCAGACTCTGTGAAGGGCCG ATTCACCATCTCCAGAGACAACGCCAAGAACTCACT GTATCTGCAAATGAACAGCCTGAGAGCCGAGGACA CGGCTGTGTATTACTGTGCGAGATCCCTCGCGGGAA ATGCTATGGACTACTGGGGCCAAGGAACCCTGGTCA CCGTCTCCTCAGGCGGAGGCGGCAGTGGCGGGGGC GGGTCCGGCGGAGGCGGGAGCGAAATTGTGTTGAC ACAGTCTCCAGCCACCCTGTCTTTGTCTCCAGGGGA AAGAGCCACCCTCTCCTGCAGGGCCAGTCAGACTAT TAGCGACTACTTACACTGGTACCAACAGAAACCTGG CCAGGCTCCCAGGCTCCTCATCAAATTTGCATCCCAA TCCATTTCTGGCATCCCAGCCAGGTTCAGTGGCAGT GGGTCTGGGACAGACTTCACTCTCACCATCAGCAGC CTAGAGCCTGAAGATTTTGCAGTTTATTACTGTCAGA ATGGTCACGGCTTTCCTCGGACGTTCGGCCAAGGGA CCAAGGTGGAAATCAAA |
| CD28 transmembrane domain | TTTTGGGTGCTGGTGGTGGTGGGCGGCGTGCTGGCC TGTTACTCCCTGCTGGTGACCGTGGCCTTCATTATCT TCTGGGTG |
| CD28 and 4-1BB costimulatory signal domain | AGGAGCAAGAGGAGCAGGCTGCTGCACAGCGACTA CATGAACATGACACCCAGGAGACCTGGCCCCACCA GAAAGCACTACCAGCCCTATGCCCCCCCCAGAGACT TTGCCGCCTACAGAAGCAGGTTCAGCGTGGTGAAG AGGGGCAGGAAGAAGCTGCTGTACATCTTCAAGCA GCCCTTCATGAGGCCCGTGCAGACCACCCAGGAGG AGGACGGCTGCAGCTGCAGGTTCCCCGAGGAGGAG GAAGGCGGATGCGAGCTG |
| N-terminal splicing domain | ACCCGGTCTGGCTACTGCCTCGACCTCAAGACCCAG GTGCAGACCCCTCAGGGCATGAAGGAGATTTCTAAC ATTCAGGTGGGCGACCTCGTGCTGAGCAACACCGGC TACAACGAGGTGCTCAACGTGTTCCCAAAGTCTAAG AAGAAGTCTTACAAGATCACACTGGAGGACGGCAA GGAGATTATTTGCTCTGAGGAGCACCTGTTCCCTACC CAGACAGGCGAGATGAACATTTCTGGCGGCCTCAAG GAGGGCATGTGCCTGTACGTGAAGGAG |
| Degrader SopE | ACCAAGATCACCCTGAGCCCCCAGAACTTCCGCATC CAGAAGCAGGAGACCACCCTGCTGAAGGAGAAGAG CACCGAGAAGAACAGCCTGGCCAAGAGCATCCTGG CCGTGAAGAACCACTTCATCGAGCTGCGCAGCAAG CTGAGCGAGCGCTTCATCAGCCACAAGAACACCGA GAGCAGCGCCACCCACTTCCACCGCGGCAGCGCCA GCGAGGGCCGCGCCGTGCTGACCAACAAGGTGGTG AAGGACTTCATGCTGCAGACCCTGAACGACATCGAC ATCCGCGGCAGCGCC |
| Degrader ERm | TCTCTGGCCCTGTCCCTCACAGCCGACCAGATGGTG TCCGCCCTCCTGGACGCCGAGCCACCAATTCTGTAC TCTGAGTACGACCCAACACGCCCTTTCAGCGAGGCC TCTATGATGGGCCTCCTCACAAACCTCGCCGACCGG GAGCTGGTGCACATGATTAACTGGGCCAAGAGAGTG CCCGGCTTCGTGGACCTCGCCCTGCACGACCAGGTG CACCTGCTGGAGTGCGCCTGGATGGAGATCCTCATG ATTGGCCTGGTGTGGCGGTCTATGGAGCACCCAGGC AAGCTGCTGTTCGCCCCTAACCTCCTGCTCGACCGC AACCAGGGCAAGTGCGTGGAGGGCGGCGTGGAGAT TTTCGACATGCTCCTCGCCACATCTAGCCGGTTCCGG ATGATGAACCTCCAAGGCGAGGAGTTCGTGTGCCTG AAGTCTATTATTCTGCTCAACTCTGGCGTGTACACCT TCCTGTCTTCTACACTCAAGTCTCTGGAGGAGAAGG ACCACATTCACCGCGTGCTCGACAAGATTACCGACA CACTCATTCACCTGATGGCCAAGGCCGGCCTCACAC TGCAACAGCAGCACCAGAGACTGGCCCAGCTGCTC CTGATCCTGTCCCACATTAGGCACATGTCTTCTAAGC GCATGGAGCACCTGTACTCTATGAAGTGCAAGAACG TGGTGCCACTGTCTGACCTGCTCCTGGAAATGCTGG ACGCCCACCGGCTG |
| Hypoxa response element | CCACAGTGCATACGTGGGCTCCAACAGGTCCTCTTC CACAGTGCATACGTGGGCTCCAACAGGTCCTCTTCC ACAGTGCATACGTGGGCTCCAACAGGTCCTCTTCCA CAGTGCATACGTGGGCTCCAACAGGTCCTCTTCCAC AGTGCATACGTGGGCTCCAACAGGTCCTCTT |
| Hypoxic condition signal response domain (oxygen-dependent degradation domain ODD) | ATGAGCGAGGACACCAGCAGCCTGTTCGACAAGCT GAAGAAGGAGCCCGACGCCCTGACCCTGCTGGCCC CTGCTGCTGGAGACACCATCATCTCCCTGGACTTCG GCAGCAACGACACCGAGACCGACGACCAGCAGCTG GAGGAGGTGCCCCTGTACAACGACGTGATGCTGCCC TCTCCCAACGAAAAACTGCAGAACATCAACCTGGCT ATGAGCCCCCTGCCCACCGCCGAAACACCAAAACC CCTGAGATCCAGCGCCGACCCCGCCCTGAACCAGG AAGTGGCCCTGAAACTGGAACCCAACCCCGAGAGC CTGGAGCTGAGCTTCACCATGCCCCAGATCCAGGAC CAGACCCCCAGCCCCAGCGACGGAAGCACCAGACA GAGCAGCCCCGAGCCTAACTCCCCCAGCGAATACTG CTTCTATGTGGACAGCGACATGGTGAACGAGTTCAA GCTGGAGCTGGTGGAAAAACTGTTCGCCGAGGACA CAGAAGCCAAAAACCCCTTCAGCACCCAGGACACA GACCTGGACCTGGAGATGCTGGCCCCCTACATCCCC ATGGACGACGACTTCCAGCTGAGATCCTTCGACCAG CTGAGCCCCCTGGAAAGCAGCAGCGCCTCCCCCGA ATCAGCCAGCCCCCAGAGCACCGTGACCGTGTTCCA G |
| C-terminal splicing domain | CTGAAAAAGATCCTGAAGATCGAGGAGCTGGACGA GCGGGAACTGATCGACATCGAAGTGTCCGGAAACC ACCTGTTCTACGCCAACGACATCCTGACACACAATA GCAGCAGCAGCGACGTG |
| CD3ζ signaling domain | AGAGTGAAATTCAGCAGAAGCGCCGACGCCCCCGC CTACCAGCAGGGACAGAATCAGCTGTACAACGAACT GAACCTGGGCAGAAGGGAGGAATACGACGTGCTGG ACAAGAGGAGAGGAAGGGACCCCGAGATGGGAGG AAAACCACAGAGAAGAAAGAACCCACAGGAGGGA CTGTACAACGAGCTGCAGAAGGACAAGATGGCCGA GGCCTACAGTGAAATTGGCATGAAGGGAGAGAGAA GAAGAGGAAAGGGACACGACGGCCTGTACCAGGGC CTGAGCACCGCTACCAAGGACACATACGACGCCCTG CACATGCAGGCCCTGCCACCAAGA |

### Example 2: Construction of lentiviral expression plasmid

Nucleic acid sequences shown in SEQ ID NOs: 7-9 were synthesized by Shanghai Generay Bioengineering Co., Ltd. and cloned into blank lentiviral expression plasmids respectively to obtain the following plasmids.

A pXW-EF1α-CD47-28BBz-P2A-EGFP recombinant plasmid carrying a nucleic acid sequence SEQ NO: 7, i.e., the traditional CD47 CAR lentiviral expression plasmid (abbreviated as pXW-CD47 CAR) recombined with a CD47-28BBz gene, was obtained by recombination with pXW-EF1α-MCS-P2A-EGFP. The nucleotide shown in SEQ ID NO: 7 coded the CD47 antigen recognition domain, the CD28 transmembrane domain, the CD28 costimulatory signal domain, the 4-1BB costimulatory signal domain, and the CD3ζ signaling domain (see Tables 1 and 2 for the specific sequences of various components).

A pXW-EF1α-CD47-28BBz-ODD-P2A-EGFP recombinant plasmid carrying a nucleic acid sequence SEQ NO: 8, i.e., a hypoxia-regulated CD47 CAR-ODD lentiviral expression plasmid fused with the oxygen-dependent degradation domain ODD (abbreviated as pXW-CD47 CAR-ODD, recombined with a CD47-28BBz-ODD gene), was obtained by recombination with pXW-EF1α-MCS-P2A-EGFP. The nucleotide shown in SEQ ID NO: 8 coded the CD47 antigen recognition domain, the CD28 transmembrane domain, the CD28 costimulatory signal domain, the 4-1BB costimulatory signal domain, the CD3ζ signaling domain and the hypoxic condition signal response domain (i.e., oxygen-dependent degradation domain ODD) (see Tables 1 and 2 for the specific sequences of various components).

A pXW-EF1α-CD47-28BB-Int^{N}-SopE-P2A-EGFP recombinant plasmid carrying a nucleic acid sequence SEQ NO: 9, i.e., a lentiviral expression plasmid (abbreviated as pXW-CD47 CAR-Int^{N}-SopE, recombined with a CD47-28BB-Int^{N}-SopE gene) of the antigen recognition unit 1, was obtained by recombination with pXW-EF1α-MCS-P2A-EGFP. The nucleotide shown in SEQ ID NO: 9 coded the CD47 antigen recognition domain, the CD28 transmembrane domain, the CD28 costimulatory signal domain, the 4-1BB costimulatory signal domain, the N-terminal splicing domain and the degrader SopE (see Tables 1 and 2 for the specific sequences of various components).

A pXW-EF1α-CD47-28BB-Int^{N}-ERm-P2A-EGFP recombinant plasmid carrying a nucleic acid sequence SEQ NO: 10, i.e., a lentiviral expression plasmid (abbreviated as pXW-CD47 CAR-Int^{N}-ERm, recombined with a CD47-28BB-Int^{N}-ERm gene) of the antigen recognition unit 2, was obtained by recombination with pXW-EF1α-MCS-P2A-EGFP. The nucleotide shown in SEQ ID NO: 10 coded the CD47 antigen recognition domain, the CD28 transmembrane domain, the CD28 costimulatory signal domain, the 4-1BB costimulatory signal domain, the N-terminal splicing domain and the degrader ERm (see Tables 1 and 2 for the specific sequences of various components).

A pXW-EF1α-BFP-HRE-ODD-Int^{C}-CD3z carrying a nucleic acid sequence SEQ NO: 11, i.e., a lentiviral expression plasmid (abbreviated as pXW-ODD-Int^{C}-CD3z, recombined with an ODD-Int^{C}-CD3z gene) of the hypoxia-regulated signal transduction unit ODD-Int^{C-}CD3z, was obtained by recombination with pXW-EF1α-BFP-MCS1-HRE-MCS2. The nucleotide shown in SEQ ID NO: 11 coded the hypoxic condition signal response domain (oxygen-dependent degradation domain ODD), the C-terminal splicing domain and the CD3ζ signaling domain (see Tables 1 and 2 for the specific sequences of various components).

A pXW-EF1α-BFP-CD47-28BB-Int^{N}-SopE-HRE-ODD-Int^{C-}CD3z carrying a nucleic acid sequence SEQ ID NO: 9 and a nucleic acid sequence SEQ NO: 11 at the same time (abbreviated as pXW-CD47 CAR-Int^{N}-SopE-HRE-ODD-Int^{C}-CD3z) was obtained by recombination with pXW-EF1α-BFP-MCS1-HRE-MCS2 (see Tables 1, 2 and 3 for the specific sequences of various components).

A pXW-EF1α-BFP-CD47-28BB-Int^{N}-ERm-HRE-ODD-Int^{C-}CD3z carrying a nucleic acid sequence SEQ NO: 10 and a nucleic acid sequence SEQ NO: 11 at the same time (abbreviated as pXW-CD47 CAR-Int^{N}-ERm-HRE-ODD-Int^{C-}CD3z) was obtained by recombination with pXW-EF1α-BFP-MCS1-HRE-MCS2 (see Tables 1, 2 and 3 for the specific sequences of various components).

The profile of each of recombinant plasmids was shown in FIG. 1.

### Example 3: Packaging, concentration and titer determination of lentivirus

### 1.1 Packaging of lentivirus

293T cell treatment: 24 h before transfection, 293T cells in the logarithmic growth phase were collected, and inoculated in a 10 cm cell culture dish (6×10⁶ to 8×10⁶ cells); and the cells grew in 10 mL of complete DMEM medium, and were cultured in a 37°C, 5% CO₂ cell incubator for 18-24 h, followed by plasmid transfection till the cell density reached 70-90%.

293T cell transfection: 1 mL of basal DMEM medium was added to a 15 mL centrifuge tube, and a transfected mixed solution was prepared from seven plasmids prepared in Example 2 according to a mass ratio of lentiviral expression plasmids: packaging plasmids (psPAX2): envelope plasmids (PMD2.G) of 1: 3: 1, with a total plasmid amount of 15 µg/dish. 30 µL of TurboFect transfection reagent was added according to a ratio of plasmids (µg): a transfection reagent (µL) of 1: 2, incubated at room temperature for 15-20 min, then added to a dish plated with 293T cells, and continued to be cultured in a 37°C, 5% CO₂ cell incubator for 48 h; a virus supernatant was then collected, and centrifuged at 1000×g, 4°C for 10 min; precipitates at the bottom of the tube were discarded; and a virus supernatant was collected.

### 1.2 Concentration of lentivirus

A 0.45 µm filter was used to further filter the viral supernatant collected by centrifugation, added with a Lenti-X lentiviral concentration reagent in 1/3 of the volume of the viral supernatant, inverted and mixed uniformly several times, incubated at 4°C overnight, and centrifuged at 2000×g, 4°C for 45 min, wherein a white precipitate was seen at the bottom of the centrifuge tube, which was concentrated virus particles. The supernatant was discarded carefully, and the white precipitate was resuspended with a blank RPMI1640 medium in 1/20 of the volume of the original virus supernatant, aliquoted in 250 µL and cryopreserved at -80°C for later use.

### 1.3 Lentiviral titer determination

Jurkat T cells were inoculated at 1×10⁵ cells/well on a 96-well U-bottom plate, and the collected lentiviral concentrate was diluted in 10-fold increments. 100 µL of virus diluent was added to the corresponding wells, added with an infection promoting agent, i.e., protamine sulfate, to adjust the concentration to 10 µg/mL, centrifuged at 1000×g, 32°C and infected for 90 min, cultured overnight followed by replacement with a fresh RPMI1640 complete medium, and continued to be cultured for 48 h; and the proportion of fluorescence-positive cells were detected by a flow cytometer. The virus titer was calculated using the following formula: Viral titer (TU/mL) = 1×105×proportion of fluorescence-positive cells / 100 × 1000 × corresponding dilution factor.

### Example 4: Preparation of genetically engineered T cells

The following lentiviral vectors were obtained by concentration in Example 3:
LU-EF1α-CD47-28BBz-P2A-EGFP (obtained from pXW-EF1α-CD47-28BBz-P2A-EGFP);
LV-EF1α-CD47-28BBz-ODD-P2A-EGFP (obtained from pXW-EF1α-CD47-28BBz-ODD-P2A-EGFP);
LV-EF1α-CD47-28BB-Int^{N}-SopE-P2A-EGFP (obtained from pXW-EF1α-CD47-28BB-Int^{N}-SopE-P2A-EGFP);
LV-EF1α-CD47-28BB-Int^{N}-ERm-P2A-EGFP (obtained from pXW-EF1α-CD47-28BB-Int^{N}-ERm-P2A-EGFP);
LV-EF1α-BFP-MCS1-HRE-MCS2 (obtained from pXW-EF1α-BFP-MCS1-HRE-MCS2);
LV-EF1α-BFP-HRE-ODD-Int^{C}-CD3z (obtained from pXW-EF1α-BFP-HRE-ODD-Int^{C}-CD3z);
LV-EF1α-BFP-CD47-28BB-Int^{N}-SopE-HRE-ODD-Int^{C}-CD3z (obtained from pXW-EF1α-BFP-CD47-28BB-Int^{N}-SopE-HRE-ODD-Int^{C}-CD3z); and
LV-EF1α-BFP-CD47-28BB-Int^{N}-ERm-HRE-ODD-Int^{C}-CD3z (obtained from pXW-EF1α-BFP-CD47-28BB-Int^{N}-ERm-HRE-ODD-Int^{C}-CD3z).

The above various lentiviral vectors were added to a 48-well flat-bottom plate plated with 1×10⁶ peripheral blood mononuclear cells, which were preactivated with an equal amount of immunomagnetic beads for three days, at MOI=5 respectively, added with an infection promoting agent, i.e., protamine sulfate, to adjust the working concentration to 10 µg/mL, centrifuged at 1000×g , 32°C and infected for 90 min, cultured overnight followed by replacement with a fresh T cell growth medium, and continued to be cultured.

The fresh T cell growth medium was added every 2-3 days, and the cell density was adjusted to 0.5×10⁶ to 2×10⁶ cells/mL. 6-7 days after infection, immunomagnetic beads of activated T cells were removed, genetically engineered T cells were continued to be cultured and expanded, and subsequent functional experiments could be performed until the cells were resting (9-14 days after removal of the beads).

### Example 5: Detection of the expression level of degrader-fused CAR molecules

In order to verify a spontaneous degradation capability of the degrader of the present invention, a plasmid transfection experiment was performed in 293T cells in this Example.

The 293T cells were plated in a 48-well plate at 5×10⁴ cells/well and transfected the next day after cell attachment. 0.5 µg of recombinant expression plasmids pXW-CD47 CAR, pXW-CD47 CAR-ODD, pXW-CD47 CAR-Int^{N}-SopE or pXW-CD47 CAR-Int^{N}-ERm were transfected respectively; a TurboFect transfection reagent was added at a ratio of the plasmids (µg): the transfection reagent (µL) of 1: 2, incubated at room temperature for 15-20 min, then added to a cell culture plate, and cultured in a 37°C, 5%CO₂ cell culture incubator for 48 h; and the expression of CAR molecules on a cell membrane was detected by flow cytometry. The results were shown in FIG. 2.

FIG. 2a showed a structure of the degrader-fused CAR, in which the degrader SopE or ERm was placed at a C-terminal, and an N-terminal splicing domain Int^{N} was connected to a signal-deficient CAR molecule and degrader.

FIG. 2b showed flow cytometry detection results of the expression levels of a positive control CD47 CAR (CD47-28BBz), an oxygen-dependent degradation domain ODD-fused CD47 CAR (CD47 CAR-ODD), and two degrader-fused CARs. The results showed that although there was no significant difference in a CAR positive rate between the groups, the expression level of the degrader SopE or ERm-fused CAR was significantly lower than that of a positive control CD47 CAR and also lower than that of CD47 CAR-ODD.

The statistical results in FIG. 2c further confirmed that the expression level of the degrader SopE or ERm-fused CAR was only 10% or 7% of that of the positive control CD47 CAR, and a spontaneous degradation rate was as high as 90% or 93%, which was also superior to a 54% degradation rate of the oxygen-dependent degradation domain ODD-fused CD47 CAR.

### Example 6: Splicing activity of condition-controlled spliceable system

The lentiviral expression plasmid constructed in Example 2 and the preparation method for the lentiviral vector in Example 3 were used to obtain a corresponding lentiviral vector.

In this Example, a lentiviral vector transduction experiment was performed in 293T cells, and the splicing activity of the condition-controlled spliceable system was tested. The above various recombinant lentiviral vectors and a no-loaded viral vector (LV-) were added individually or mixed at MOI=5 to a 6-well flat-bottom plate plated with 1×10⁶ 293T cells, respectively; an infection-promoting reagent (Polybrene) was added and adjusted for a working concentration to 10 µg/mL, and cultured overnight; and then a fresh cell growth medium was replaced and continued to be cultured to obtain nine different genetically engineered 293T cells that stably integrate: 1) EF1α-CD47-28BBz-P2A-EGFP; 2) EF1α-CD47-28BB-Int^{N}-SopE-P2A-EGFP; 3) EF1α-CD47-28BB-Int^{N}-ERm-P2A-EGFP; 4) EF1α-CD47-28BBz-P2A-EGFP and hypoxia-regulated signaling unit EF1α-BFP-HRE-ODD-Int^{C}-CD3z; 5) EF1α-CD47-28BB-Int^{N}-SopE-P2A-EGFP and hypoxia-regulated signaling unit EF1α-BFP-HRE-ODD-Int^{C}-CD3z; 6) EF1α-CD47-28BB-Int^{N}-ERm-P2A-EGFP and hypoxia-regulated signaling unit EF1α-BFP-HRE-ODD-Int^{C}-CD3z; 7) EF1α-CD47-28BBz-P2A-EGFP and EF1α-BFP-MCS1-HRE-MCS2 (Mock); 8) EF1α-CD47-28BB-Int^{N}-SopE-P2A-EGFP and EF1α-BFP-MCS1-HRE-MCS2 (Mock); and 9) EF1α-CD47-28BB-Int^{N}-ERm-P2A-EGFP and EF1α-BFP-MCS1-HRE-MCS2 (Mock). The above nine genetically engineered T cells were cultured under a hypoxic condition (1% O₂) for 24 h; the cells were collected after the culture and eluted with FACS buffer once, added with a 2 µg/mL flow cytometry antibody PE-anti-DYKDDDDK, incubated at room temperature in the dark for 20 min, eluted twice with FACS buffer after the incubation, and resuspended and mixed uniformly with 300 µL of FACS buffer; and then, the expression of CAR molecules was detected by a flow cytometer. In addition, the six genetically engineered 293T cells, which stably integrate: 1) EF1α-CD47-28BBz-P2A-EGFP, 2) EF1α-CD47-28BB-Int^{N}-SopE-P2A-EGFP, 3) EF1α-CD47-28BB-Int^{N}-ERm-P2A-EGFP, 4) EF1α-CD47-28BBz-P2A-EGFP and hypoxia-regulated signaling unit EF1α-BFP-HRE-ODD-Int^{C}-CD3z, 5) EF1α-CD47-28BB-Int^{N}-SopE-P2A-EGFP and hypoxia-regulated signaling unit EF1α-BFP-HRE-ODD-Int^{C}-CD3z, and 6) EF1α-CD47-28BB-Int^{N}-ERm-P2A-EGFP and hypoxia-regulated signaling unit EF1α-BFP-HRE-ODD-Int^{C}-CD3z, were cultured under a normoxic condition (21% O₂) and a hypoxic condition (1% O₂) for 24 h, respectively, and after culture, the cells were collected for western blotting. The results were shown in FIG. 3.

FIG. 3a showed structures and compositions of a degrader-fused CAR and a hypoxia-regulated signaling unit ODD-Int^{C}-CD3z, wherein ODD-Int^{C}-CD3z was composed of the oxygen-dependent degradation domain ODD, the C-terminal splicing domain Int^{C}, and the signal domain CD3z.

The flow cytometry detection result in FIG. 3b showed that the CAR expression levels of engineered cells that integrate both the degrader-fused CAR and the hypoxia-regulated signaling unit ODD-Int^{C}-CD3z were further improved under a hypoxic condition, suggesting that the hypoxia-regulated signaling unit ODD-Int^{C}-CD3z was enriched under the hypoxic condition and achieving splicing of the degrader-fused CAR to release degraders.

FIG. 3c showed an induction fold of the CAR expression level after splicing, and the induction fold of the degrader SopE or ERm-fused CAR was 4 or 5-fold respectively, while there was no change in the expression level of a control CD47 CAR.

A Western Blotting result in FIG. 3d confirmed that the degrader-fused CAR and the signaling unit were successfully spliced with an efficiency of up to 100% under a hypoxic condition, and complete splicing was achieved under a hypoxic condition (1% O₂), thereby obtaining a single band with a molecular weight smaller than that of an unspliced molecule.

### Example 7: Expression of chimeric antigen receptor regulated by condition-controlled spliceable system

By using the lentiviral expression plasmid constructed in Example 2, the preparation method for the lentiviral vector in Example 3 and the preparation method for the genetically engineered T cells described in Example 4, five engineered T cells, which stably integrate EF1α-CD47-28BBz-P2A-EGFP, EF1α-CD47-28BB-Int^{N}-SopE-P2A-EGFP, EF1α-BFP-CD47-28BB-Int^{N}-SopE-HRE-ODD-Int^{C}-CD3z, EF1α-CD47-28BB-Int^{N}-ERm-P2A-EGFP or EF1α-BFP-CD47-28BB-Int^{N}-ERm-HRE-ODD-Int^{C}-CD3z genes were prepared. The above five genetically engineered T cells were cultured under a normoxic condition (21% O₂) or a hypoxic condition (1% O₂) for 24 h respectively; the cells were collected after the culture and eluted with FACS buffer once, added with a 2 µg/mL flow cytometry antibody PE-anti-DYKDDDDK, incubated at room temperature in the dark for 20 min, eluted twice with FACS buffer after the incubation, and resuspended and mixed uniformly with 300 µL of FACS buffer; and then, the expression of HER2 CAR molecules was detected by the flow cytometer. The results were shown in FIG. 4.

The flow cytometry detection results in FIG. 4a showed that CD47 CAR molecules were highly expressed by positive control CD47 CAR-T cells (CD47-28BBz) under both normoxic and hypoxic conditions, while CAR molecules regulated by the condition-controlled spliceable system were lowly expressed in a normoxic environment, but induction-expressed in a hypoxic environment, especially for the engineered T cells stably integrating EF1α-BFP-CD47-28BB-Int^{N}-SopE-HRE-ODD-Int^{C}-CD3z.

The statistical results in FIG. 4b confirmed that a positive rate of the degrader SopE or ERm-fused CAR was 46% or 24% of that of the positive control under a normoxic condition, respectively, and the expression level was reduced to 4% or 2% of that of the control, but was significantly up-regulated under a hypoxic condition; and the induction fold was 9- or 3-fold, respectively, indicating that the condition-controlled spliceable system based on the degrader SopE was more active.

### Example 8: Tumor cell killing of chimeric antigen receptor T cells regulated by condition-controlled spliceable system

The tumor cell killing efficiency was evaluated by a luciferase-based cytotoxicity assay. First, 1×10⁴ SKOV3-Luc (firefly luciferase gene-modified human ovarian cancer cells) or NCI-H292-Luc (firefly luciferase gene-modified human lung cancer cells) were inoculated on a 96-well flat-bottom black plate with 100 µL of medium per well, and cultured in a 37°C, 5% CO₂ cell incubator for 18 h. On the second day, the genetically engineered T cells prepared in Example 7 and non-transduced T cells cultured at the same time were added to the wells containing target cells at a ratio of effector cells : target cells of 1: 4 (0.25), 1: 2 (0.5), 1: 1 (1) and 2: 1 (2), and were cultured under a normoxic condition (21% O₂) or a hypoxic condition (1% O₂) for 24 h, respectively; and a luciferase activity value of the target cells was detected by a GloMax^{®}96 microplate luminescence detector after co-culture. The results were shown in FIG. 5.

A formula for calculating a cell killing rate was as follows: Cell killing rate (%) = (luciferase activity value of non-transduced T cell group - luciferase activity value of experimental group)/ luciferase activity value of non-transduced T cell group × 100.

CD47 CAR-T cells (positive control) can effectively kill SKOV3 (FIG. 5a) and NCI-H292 tumor cells (FIG. 5B) under either normoxic or hypoxic condition, and had no selective killing properties.

The tumor killing activity of the CD47 CAR-T cells regulated by the condition-controlled spliceable system was low under the normoxic condition, and the killing rates of the CD47 CAR-T cells regulated by a SopE-based condition-controlled spliceable system were 3% (SKOV3) and 6% (NCI-H292) at a ratio of effector cells : target cells of 1: 1, while SKOV3 tumor cells (84%) and NCI-H292 tumor cells (96%) can be selectively and efficiently killed under the hypoxic condition; and the killing activities against the tumor cells were increased by factors of 28 and 16. The killing rates of the CD47 CAR-T cells regulated by an ERm-based condition-controlled spliceable system were 3% (SKOV3) and 6% (NCI-H292) at a ratio of effector cells : target cells of 1: 1, while SKOV3 tumor cells (58%) and NCI-H292 tumor cells (96%) can be selectively and efficiently killed under the hypoxic condition; and the killing activities against the tumor cells were increased by factors of 19 and 16.

### Example 9: In-vivo antitumor effects of CAR-T cells regulated by condition-controlled spliceable system

Local hair removal on the back of B-NDG mice raised in a sterile isolator was performed with a hair removal cream or an animal shaver one day in advance, such that the skin of a tumor cell inoculation site was exposed. Each mouse was fixed with the left hand, that is, the head, neck and back skin of the mouse were grasped with the left hand at the same time; and after a part to be hair-removed on the right side of the back was fully exposed by turning its back to the left, this part was disinfected by an alcohol cotton ball with the right hand. A 1 mL insulin syringe was used to blow and mix uniformly the preprepared NCI-H292 tumor cells, and then suck 125 µL of cell suspension (5×10⁶ tumor cells), and a tip of a needle was pierced subcutaneously and diagonally into the mouse at an angle of 30°-40° from the skin; and the cell suspension was slowly injected to avoid cell spillage. After the injection of 125 µL of cell suspension was completed, the needle was left for 2-3 seconds and then quickly withdrawn, and a clearly visible bulge could be seen under the skin at the injection site. After cell inoculation, the tumorigenesis and health status of the mice were observed every 2-3 days, and a baseline tumor volume was measured with a vernier caliper after tumorigenesis, and subsequent experiments were carried out.

On the fifth and eleventh days after tumor cell inoculation, 5×10⁶ CAR-T cells regulated by the condition-controlled spliceable system and negative control T cells (UTDs) were injected intravenously or intratumorally, respectively; and tumor sizes were measured every 2-3 days, and long and short diameters of tumors were measured with the vernier caliper, respectively. The results were shown in FIG. 6.

A calculation formula of a tumor volume was as follows: volume = (long diameter × short diameter²)/2.

The growth of lung cancer can be effectively controlled by intravenous infusion and intratumoral injection of CAR-T cells regulated by condition-controlled spliceable systems. Compared with a UTD treatment group, the CAR-T cells (stably integrating EF1α-BFP-CD47-28BB-Int^{N}-SopE-HRE-ODD-Int^{C}-CD3z or EF1α-BFP-CD47-28BB-Int^{N}-ERm-HRE-ODD-Int^{C}-CD3z) regulated by a degrader SopE-based condition-controlled spliceable system had a more significant tumor inhibition effect; and the tumor suppression rates were 72.1% (intravenous infusion) and 85.6% (intratumoral injection) at one month after cell reinfusion, respectively.

The foregoing is only preferred embodiments of the present invention and is not intended to limit the present invention in any form. Although the present invention has been revealed as above with a preferred embodiment, it is not used to limit the present invention. A person skilled in the art, without departing from the scope of the technical solution of the present invention, may make some changes or modifications to obtain the equivalent embodiments of the equivalent changes by using the above disclosed technical content, but any amendments, equivalent changes and modifications made to the above embodiments according to the technical essence of the present invention without departing from the content of the technical solution of the present invention still fall within the scope of the technical solution of the present invention.

## Claims

1. A condition-controlled spliceable chimeric antigen receptor molecule, comprising an antigen recognition unit and a signal transduction unit, wherein the antigen recognition unit comprises an antigen recognition domain, a transmembrane domain, a costimulatory signal domain, an N-terminal splicing domain, and a degrader; and the signal transduction unit comprises a conditional signal response domain, a C-terminal splicing domain, and a signaling domain.

2. The chimeric antigen receptor molecule according to claim 1, wherein an antigen to which the antigen recognition domain binds is one or more selected from CD47, AXL, EGFR, CD7, CD24, FAP, CD147, HER2, ROR1, ROR2, CD133, EphA2, CD171, CEA, EpCAM, TAG72, IL-13Rα, EGFRvIII, GD2, FRα, PSCA, PSMA, GPC3, CAIX, Claudin182, VEGFR2, PD-L1, MSLN, MUC1, c-Met, B7-H3 or TROP2 antigen; preferably, the antigen to which the antigen recognition domain binds is the CD47 antigen;
preferably, the transmembrane domain is one or more selected from CD3ζ, CD4, CD8, CD28 or CD137/4-1BB transmembrane domain; more preferably, the transmembrane domain is the CD28 transmembrane domain;
preferably, the costimulatory domain in the antigen recognition unit is one or more selected from CD2, CD27, CD28, CD40, OX40, CD137/4-1BB, TLR1, TLR2, TLR3, TLR4, TLR5, TLR6, TLR7, TLR8, TLR9, TLR10, TLR11 or Dap10 costimulatory domain; more preferably, the costimulatory domain in the antigen recognition unit is one or more selected from the CD28 or CD137/4-1BB costimulatory domain;
preferably, the N-terminal splicing domain is one or more selected from a protein intron or a SpyTag/SpyCatcher self-assembler; more preferably, the N-terminal splicing domain is a protein intron Int^{N}; and
preferably, the degrader is one or more selected from a dihydrofolate reductase (DHFR), an estrogen receptor (ER), a Salmonella type III secretion system effector protein (SopE), a plant hormone-inducible protein degrader, an unstable domain (AD) or a photosensitive protein degrader; more preferably, the degrader is one or more selected from an estrogen receptor or a Salmonella type III secretion system effector protein; and most preferably, the degrader is a mutant estrogen receptor (ERm).

3. The chimeric antigen receptor molecule according to claim 1 or 2, wherein the conditional signal response domain is one or more selected from an oxygen-dependent degradation domain (ODD), a temperature-sensitive domain, a pH-sensitive domain, a photosensitive domain or an inflammatory cytokine response domain; preferably, the conditional signal response domain is the oxygen-dependent degradation domain;
preferably, the C-terminal splicing domain is one or more selected from a protein intron or a SpyTag/SpyCatcher self-assembler; more preferably, the C-terminal splicing domain is a protein intron Int^{C}; and
preferably, the signaling domain is one or more selected from a CD3ξ, FcyRIII, FcεRI or Fc receptor signaling domain or an immunoreceptor tyrosine-based activation motif (ITAM)-carrying signaling molecule; and more preferably, the signaling domain is a CD3ξ signaling domain.

4. The chimeric antigen receptor molecule according to claim 3, wherein the signal transduction unit further comprises a costimulatory signal domain; and
preferably, the costimulatory signal domain in the signal transduction unit is one or more selected from CD2, CD27, CD28, CD40, OX40, CD137/4-1BB, TLR1, TLR2, TLR3, TLR4, TLR5, TLR6, TLR7, TLR8, TLR9, TLR10, TLR11 or Dap10 costimulatory signal domain.

5. The chimeric antigen receptor molecule according to any one of claims 1 to 4, wherein an amino acid sequence of the antigen recognition unit is shown in SEQ NO: 1 or SEQ NO: 2; and/or an amino acid sequence of the signal transduction unit is shown in SEQ NO: 3.

6. A nucleic acid molecule coding the chimeric antigen receptor molecule according to any one of claims 1 to 5, wherein
preferably, in the nucleic acid molecule, a nucleotide sequence coding the signal transduction unit also comprises a nucleotide sequence coding a conditional signal response element;
preferably, the conditional signal response element is one or more selected from a hypoxia response element (HRE), a temperature-sensitive element, a pH-sensitive element, a photosensitive element or an inflammatory cytokine response element; more preferably, the conditional signal response element is the hypoxia response element (HRE);
preferably, a nucleotide sequence coding the signal transduction unit is shown in SEQ NO: 4; and
preferably, a nucleotide sequence of the nucleic acid molecule is shown in SEQ NO: 5 or 6.

7. A vector comprising the nucleic acid molecule according to claim 6, wherein
preferably, the vector is one or more selected from a plasmid, a retroviral vector, a lentiviral vector, an adenoviral vector, an adeno-associated viral vector, a vaccinia virus vector, a herpes simplex virus vector, a forest encephalitis virus vector, a poliovirus vector, a Newcastle disease virus vector or a transposon; and more preferably, the vector is the lentiviral vector.

8. A genetically engineered host cell comprising the exogenous nucleic acid molecule according to claim 6 that is integrated in its chromosome, or comprising the vector according to claim 7, wherein
preferably, the host cell is one or more selected from an isolated human-derived cell or a genetically engineered immune cell;
further preferably, the isolated human-derived cell is one or more selected from an embryonic stem cell, an umbilical cord blood-derived stem cell, an induced pluripotent stem cell, a hematopoietic stem cell, a mesenchymal stem cell, an adipose-derived stem cell, a T cell, an NK cell, an NKT cell or a macrophage;
further preferably, the genetically engineered immune cell is one or more selected from a genetically engineered T cell, NK cell, NKT cell or macrophage; more preferably, the genetically engineered immune cell is the genetically engineered T cell;
more preferably, the genetically engineered immune cell is one or more selected from a chimeric antigen receptor T cell (CAR-T cell), a chimeric antigen receptor NK cell (CAR-NK cell), a chimeric antigen receptor NKT cell (CAR-NKT cell), a chimeric antigen receptor macrophage (CAR-mø) or a T cell receptor T cell (TCR-T cell); and
most preferably, the genetically engineered immune cell is the chimeric antigen receptor T cell.

9. A method for preparing the genetically engineered host cell according to claim 8, comprising: introducing into the host cell the nucleic acid molecule according to claim 6 and/or the vector according to claim 7; and
preferably, the introduction is transfection or transduction.

10. A method for cellular immunotherapy comprising:
introducing into a host cell the nucleic acid molecule according to claim 6 and/or the vector according to claim 7, and administrating to a subject in need thereof a therapeutically effective amount of the host cell; or
administrating to the subject in need thereof a therapeutically effective amount of the genetically engineered host cell according to claim 8, wherein
preferably, the introduction is performed simultaneously, in a chronological order, or in sequence;and
preferably, the method for cellular immunotherapy is useful for treating a hypoxic disease; more preferably, the hypoxic disease is a cancer; and further preferably, the cancer is a solid tumor, such as one or more of neuroblastoma, lung cancer, breast cancer, esophageal cancer, gastric cancer, liver cancer, cervical cancer, ovarian cancer, kidney cancer, pancreatic cancer, nasopharyngeal cancer, small bowel cancer, large bowel cancer, colorectal cancer, bladder cancer, bone cancer, prostate cancer, thyroid cancer or brain cancer.
